# EUROPÄISCHE PATENTANMELDUNG

(11) **EP 2 484 392 A1**
(43) Veröffentlichungstag der Anmeldung: **08.08.2012**
(21) Anmeldenummer: 12166241.5
(22) Anmeldetag: 08.07.2003
(51) Int. Cl.: A61M 5/168, A61M 5/145, A61M 5/142

(54) **Infusionspumpe zur dosierten Verabreichung einer medizinischen Flüssigkeit**

(30) Priorität: 24.07.2002 DE 10233622
(62) Teilanmeldung aus: 03732167.6
(71) Anmelder: F. Hoffmann-La Roche AG, 4070 Basel (CH); Roche Diagnostics GmbH, 68305 Mannheim (DE)
(72) Erfinder: Sidler, Rudolf, 4513 Langendorf (CH)
(74) Vertreter: Schwabe - Sandmair - Marx

(57) **Zusammenfassung**

Infusionspumpe zur dosierten Verabreichung einer zu injizierenden medizinischen Flüssigkeit, umfassend
a) einen Flüssigkeitsbehälter (1) zur Aufbewahrung der Flüssigkeit,
b) ein Flüssigkeitsfördermittel (2) zum Fördern der Flüssigkeit aus dem die zu injizierende Flüssigkeit enthaltenden Flüssigkeitsbehälter (1)
c) und eine Ausschüttkontrollvorrichtung (3) zur Dosierung der Ausschüttung der Flüssigkeit,
d) wobei das Flüssigkeitsfördermittel (2) die in dem Flüssigkeitsbehälter (1) aufbewahrte Flüssigkeit mit Druck beaufschlagt, so dass die Flüssigkeit von einer Ausschüttöffnung (8) des Flüssigkeitsbehälters (1) zu einem Einlass (9) der Ausschüttkontrollvorrichtung (3) gefördert wird,
e) das Flüssigkeitsfördermittel (2) die Flüssigkeit am Einlass (9) der Ausschüttkontrollvorrichtung (3) mit Druck beaufschlagt,
f) das Flüssigkeitsfordermittel (2) von der Ausschüttkontrollvorrichtung (3) entkoppelt ist,
g) der Flüssigkeitsbehälter (1) einen Kolbenstopfen (6) umfasst, der die Flüssigkeit bei seinem Vortrieb ausschüttet,
h) und das Flüssigkeitsfördermittel (2) den Kolbenstopfen (6) vortreibt.

## Beschreibung

Die Erfindung betrifft eine Infusionspumpe zur dosierten Verabreichung einer zu injizierenden medizinischen Flüssigkeit, insbesondere zur kontrollierten Abgabe von Mikrodosen einer einen medizinischen oder therapeutischen Wirkstoff enthaltenden Flüssigkeit über einen längeren Zeitraum, beispielsweise zur Langzeitabgabe von Insulin oder dergleichen. Ferner betrifft die Erfindung ein Verfahren zum Steuern einer solchen dosierten Verabreichung, ein Steuerprogramm hierfür und ein Halbleiterbauelement, umfassend ein solches Steuerprogramm zur Verwendung in einer Infusionspumpe.

Aus dem Stand der Technik sind Infusionsvorrichtungen bekannt, um eine einen medizinischen oder therapeutischen Wirkstoff enthaltende Flüssigkeit über einen längeren Zeitraum durch Injektion in ein Körpervolumen, beispielsweise Venenvolumen oder Gewebe, zu verabreichen. Ein wichtiger Parameter ist dabei die pro Zeiteinheit zu verabreichende Flüssigkeitsmenge, aus der sich der verabreichte Wirkstoff errechnen lässt. In Spitälern werden üblicherweise oberhalb der Injektionsstelle aufgehängte Infusionsflaschen eingesetzt, von wo aus die Flüssigkeit aufgrund der Schwerkraft über eine als Ausschüttkontrollvorrichtung dienende variable Schlauchklemme abgegeben wird. Infusionsflaschen eignen sich nicht als mobile Infusionsvorrichtungen, insbesondere nicht zur Langzeitabgabe von vergleichsweise geringeren Dosen.

Aus dem Stand der Technik sind auch Infusionspumpen bekannt, deren Betriebsweise so gesteuert wird, dass die Menge der verabreichten Flüssigkeit der gewünschten Dosierung entspricht. Solche Infusionspumpen umfassen einen Medikamentenbehälter, aus dem die Flüssigkeit durch Vortreiben eines Kolbenstopfens ausgeschüttet wird. Die Dosierung erfolgt durch Steuerung des Kolbenvortriebs. Zur Langzeitabgabe von vergleichsweise geringen Dosen kann ein Untersetzungsgetriebe, beispielsweise eine Zahnstange, zum Vortrieb des Kolbenstopfens vorgesehen sein. Solchen Infusionspumpen ist gemeinsam, dass die Genauigkeit der Dosierung durch Fertigungstoleranzen der Infusionspumpe selbst vorgegeben ist. Dies erfordert die Einhaltung enger Fertigungstoleranzen, was bedingt, dass solche Infusionspumpen üblicherweise nur für einen Typ von Medikamentenbehälter, beispielsweise Ampulle, geeignet sind.

US 6,348,043 B1 offenbart eine Infusionsvorrichtung, bei der eine medizinische Flüssigkeit aus einer Ampulle durch Vortreiben eines als Flüssigkeitsfördermittel dienenden Kolbenstopfens ausgeschüttet wird. Eine Membran auf der Stirnfläche des Kolbenstopfens wird durch eine Druckfeder mit Druck beaufschlagt, so dass die Flüssigkeit auch im Anschluss an eine Vortriebsbewegung des Kolbenstopfens langsam ausgeschüttet wird. Ein als Anschlag dienendes Treppenglied dient zur Vorgabe der Dosierung und muss zur Verabreichung von vergleichsweise kleinen Dosen präzise gefertigt sein. Bei dieser Vorrichtung, wie auch bei den vorgenannten Infusionspumpen, sind die Funktionen eines Flüssigkeitsausstosses aufgrund des Kolbenvortriebs und einer Dosierung nicht voneinander entkoppelt, was die vorgenannten Beschränkungen hinsichtlich der Dosiergenauigkeit und der Flexibilität bedingt.

Es ist eine Aufgabe der Erfindung, eine Infusionspumpe zu schaffen, mit der sich in einfacher Weise eine genaue und flexible Dosierung der zu injizierenden Flüssigkeit erzielen lässt. Insbesondere soll eine mobile Infusionspumpe zur Langzeitabgabe von vergleichsweise geringen Dosen einer zu injizierenden Flüssigkeit geschaffen werden. Ferner sollen ein Steuerverfahren, ein Steuerprogramm und ein Halbleiterbauelement mit einem solchen Steuerprogramm für die vorgenannten Zwecke bereitgestellt werden.

Diese Aufgabe wird gelöst durch eine Infusionspumpe mit den Merkmalen nach Anspruch 1, durch ein Steuerverfahren mit den Merkmalen nach Anspruch 17, durch ein Steuerprogramm nach Anspruch 18 sowie durch ein Halbleiterbauelement nach Anspruch 19 zum Speichern des Steuerprogramms. Vorteilhafte Weiterbildungen sind Gegenstand der rückbezogenen Unteransprüche.

Eine Infusionspumpe gemäß der Erfindung umfasst ein Flüssigkeitsfördermittel zum Fördern der Flüssigkeit aus einem Flüssigkeitsbehälter, der die zu injizierende Flüssigkeit enthält, sowie eine Ausschüttkontrollvorrichtung zur Dosierung der Ausschüttung der Flüssigkeit. Erfindungsgemäß beaufschlagt das Flüssigkeitsfördermittel die Flüssigkeit an einem Einlass der Ausschüttkontrollvorrichtung zumindest unmittelbar vor und während der Ausschüttung mit einem Druck und ist das Flüssigkeitfördermittel von der Ausschüttkontrollvorrichtung entkoppelt. Die Ausschüttkontrollvorrichtung ist im Strom der Flüssigkeit dem Flüssigkeitsfördermittel nachgeschaltet. Sowohl das Flüssigkeitsfördermittel als auch die Ausschüttkontrollvorrichtung wirken auf die Flüssigkeit, letztere stromabwärts von dem Fördermittel. Vorteilhafterweise besteht zwischen dem Fördermittel und der Ausschüttkontrollvorrichtung kein mechanischer Eingriff, der die Fördertätigkeit in Abhängigkeit von der Dosierung steuert oder regelt; vorzugsweise besteht keinerlei mechanischer Eingriff.

Erfindungsgemäß dient somit das Flüssigkeitsfördermittel ausschließlich der Förderung der Flüssigkeit, beispielsweise durch Ausstoßen aus einer Ampulle, dient dieses jedoch nicht der Ausschüttkontrolle bzw. der Dosierung der Ausschüttung der Flüssigkeit. Vorteilhaft ist, dass das Flüssigkeitsfördermittel einfach und kostengünstig ausgelegt werden kann, weil die Genauigkeit der Dosierung nicht durch das Fördermittel, sondern nur durch die Auschüttkontrollvorrichtung bestimmt ist. Vorteilhafterweise kann auf die Verwendung von mechanischen Präzisionsbauteilen für das Fördermittel verzichtet werden. Ferner kann das Flüssigkeitsfördermittel insgesamt flexibler ausgelegt werden, beispielsweise zur Verwendung verschiedener Typen von Flüssigkeitsbehältern, weil eine präzise Abstimmung von Fördermittel, beispielsweise Gewindestangenantrieb für einen Kolbenstopfen, und Flüssigkeitsbehälter, beispielsweise Ampulle mit einem solchen Stopfen, nicht mehr notwendig ist.

Erfindungsgemäß wird die Menge der ausgeschütteten Flüssigkeit, die aufgrund der Druckbeaufschlagung von dem Flüssigkeitsfördermittel zu der Ausschüttkontrollvorrichtung gefördert wird, von der Ausschüttkontrollvorrichtung kontrolliert. Vorteilhaft ist, dass aufgrund der Entkopplung von Fördermittel und Ausschüttkontrollvorrichtung letztgenannte für verschiedene Typen von Fördermitteln und/oder Flüssigkeitsbehältern verwendet werden kann, was die Flexibilität der Infusionspumpe noch weiter erhöht.

Mit der Entkopplung der Funktionen der Förderdruckerzeugung und der Dosierung, nämlich durch die in Strörnungsrichtung der Förderdruckerzeugung nachgeordnete Dosierung mittels der Ausschüttkontrollvorrichtung, wird die Sicherheit erhöht, dass tatsächlich auch nur die gewünschte Flüssigkeitsmenge ausgeschüttet wird. Bei herkömmlichen Infusionspumpen, bei denen das Flüssigkeitsfördermittel gleichzeitig auch dosiert, kann es aufgrund von Wärmeausdehnungen der Flüssigkeit zu Fehlausschüttungen kommen. Bei der erfindungsgemäßen Infusionspumpe hingegen mit nachgeschalteter Ausschüttkontrollvorrichtung erhöht sich im Falle einer Wärmeausdehnung der Druck stromaufwärts von dem Einlass der Ausschüttkontrollvorrichtung. Gegebenenfalls gibt das Fördermittel nach und kompensiert so die Druckerhöhung ganz oder teilweise. Nur ein Flüssigkeitsvolumen stromabwärts von dem Einlass der Ausschüttkontrollvorrichtung führt durch seine Wärmeausdehnung zu einer Fehlausschüttung, die aber aufgrund des verringerten Volumens gegenüber herkömmlichen Infusionspumpen entsprechend verringert ist. Die Erfindung reduziert somit auf Temperaturschwankungen beruhende Fehlausschüttungen.

Das Flüssigkeitsfördermittel beaufschlagt die Flüssigkeit am Einlass der Ausschüttkontrollvorrichtung zumindest unmittelbar vor und während der Ausschüttung der Flüssigkeit durch die Ausschüttkontrollvorrichtung. Gemäß einer vorteilhaft einfachen Ausführungsform wird die Flüssigkeit am Einlass der Ausschüttkontrollvorrichtung permanent mit Druck beaufschlagt, so dass zusätzliche Steuermittel zur Steuerung der Druckbeaufschlagung nicht vorgesehen werden müssen.

Bevorzugt öffnet die Ausschüttkontrollvorrichtung einen mit deren Einlass kommunizierenden Auslass vorübergehend, so dass während eines vorgebbaren Zeitintervalls druckbeaufschlagte Flüssigkeit kontrolliert ausgeschüttet wird. Zur Steuerung der Ausschüttkontrollvorrichtung ist bevorzugt ein elektronisches Steuermittel vorgesehen, um die Dauer des Zeitintervalls variabel in Entsprechung zu der zu erzielenden Dosierung zu steuern.

Gemäß einer bevorzugten Ausführungsform ist ein Flüssigkeitsbehälter zur Aufbewahrung der Flüssigkeit mit der Infusionspumpe verbunden, oder gar in diese integriert, so dass eine mobile Infusionspumpe bereitgestellt werden kann. Gemäß dieser Ausführungsform beaufschlagt das Flüssigkeitsfördermittel die in dem Flüssigkeitsbehälter aufbewahrte Flüssigkeit mit Druck, ganz besonders bevorzugt permanent mit Druck, um die Flüssigkeit von einer Ausschüttöffnung des Flüssigkeitsbehälters zu dem Einlass der Ausschüttkontrollvorrichtung zu fördern. Bevorzugt ist der Flüssigkeitsbehälter über eine vergleichsweise kurze und wenig oder nicht nachgiebige Leitung mit der Ausschüttkontrollvorrichtung verbunden, so dass der Druck allenfalls zu einer vernachlässigbaren Verformung der Wandungen der Leitung führt. Die Ausschüttkontrollvorrichtung kann auch unmittelbar an oder in der Ausschüttöffnung des Flüssigkeitsbehälters vorgesehen, beispielsweise in diese integriert, sein.

Ganz besonders bevorzugt beaufschlagt das Flüssigkeitsfördermittel den Flüssigkeitsbehälter permanent mit Druck, so dass die Flüssigkeit unkontrolliert zu der Ausschüttkontrollvorrichtung gefördert wird und die Ausschüttung ausschließlich durch die zu diesem Zweck von dem Flüssigkeitsfördermittel entkoppelte Ausschüttkontrollvorrichtung kontrolliert wird.

Der Flüssigkeitsbehälter kann eine übliche Glas- oder Kunststoffampulle, beispielsweise mit im Wesentlichen zylindrischem Querschnitt, oder ein im Wesentlichen rechteckiger Körper sein, der einen Kolbenstopfen umfasst, der die Flüssigkeit aus dem Behälter ausschüttet, wenn der Kolbenstopfen von dem Flüssigkeitsfördermittel vorgetrieben wird. Der Flüssigkeitsbehälter kann auch ein Beutel oder dergleichen sein, dessen Wandung durch Druckbeaufschlagung verformt wird, um Flüssigkeit auszuschütten. Bevorzugt ist die Ausschüttöffnung an einem im Wesentlichen dem Flüssigkeitsfördermittel gegenüberliegenden Ende des Flüssigkeitsbehälters angeordnet, so dass verteilhaft niedrige Kräfte die Ausschüttung der Flüssigkeit bewirken können.

Bevorzugt umfasst das Flüssigkeitsfördermittel ein Antriebsmittel, das den Kolbenstopfen zu der Ausschüttöffnung hin mit einer Antriebskraft beaufschlagt. Bevorzugt ist das Antriebsmittel elastisch vorgespannt und beaufschlagt den Kolbenstopfen zum Vortrieb permanent mit einer Antriebselastizitätskraft, beispielsweise mittels einer vorgespannten Druckfeder, eines Spiralfedermechanismus, eines Drehfedermechanismus, eines in einer Ausgangsstellung komprimierten Elastomers oder eines Druckgasreservoirs, beispielsweise einer aufpumpbaren Pneumatik. Solange die Ausschüttkontrollvorrichtung eine geschlossene Stellung, d.h. eine Sperrstellung, einnimmt, um die Ausschüttung der Flüssigkeit zu unterbinden, kann der Kolbenstopfen nicht vorgetrieben werden, weil die in dem Flüssigkeitsbehälter aufbewahrte Flüssigkeit im Wesentlichen inkompressibel ist. Gibt hingegen die Ausschüttkontronvorrichtung eine Flüssigkeitsausschüttung frei, so ist die Durchflussrate im Wesentlichen durch die bevorzugt voreinstellbare Elastizitätskraft des Antriebsmittels und den dadurch bedingten Vortrieb des Kolbenstopfens in Kombination mit der Größe des Strömungsquerschnitts und/oder der Öffnungszeit des Strömungsquerschnitts der Ausschüttkontrollvorrichtung vorgegeben, so dass sich die tatsächlich verabreichte Dosis in einfacher Weise unter Berücksichtigung der in der Flüssigkeit enthaltenen Konzentration des medizinischen oder therapeutischen Wirkstoffs errechnen lässt. Diese Berechnung kann mit Hilfe eines üblichen elektronischen Steuermittels, beispielsweise eines Mikroprozessors, vorgenommen werden, das vorteilhafterweise Bestandteil der Infusionspumpe ist.

Das Flüssigkeitsfördermittel kann auch eines sein, dass die Flüssigkeit am Einlass der Ausschüttkontrollvorrichtung nur intermittierend mit Druck beaufschlagt, also zumindest vor und während der Betätigung der Ausschüttkontrollvorrichtung, so dass sich der Energieverbrauch der Infusionspumpe vorteilhaft herabsetzen lässt. Eine solche Ausführungsform ist insbesondere dann von Vorteil, wenn ein motorgetriebenes Flüssigkeitsfördermittel verwendet wird, beispielsweise ein motorgetriebener Kolbenstopfenvortriebsmechanismus.

Bevorzugt kann der Druck, mit dem die Flüssigkeit am Einlass der Ausschüttkontrollvorrichtung beaufschlagt wird, variiert werden, was in bekannter Weise zu einer Änderung der Durchflussrate und damit der tatsächlich verabreichten Dosierung führt.

Bei sämtlichen vorstehenden Ausführungsformen schließt die Ausschüttkontrollvorrichtung bevorzugt permanent und wird deren Auslass nur vorübergehend freigegeben, um die Ausschüttung einer dosierten Flüssigkeitsmenge zu bewirken. Bevorzugt ist der Querschnitt des Auslasses der Ausschüttkontrollvorrichtung variabel vorgebbar. Zur Steuerung der Ausschüttkontrollvorrichtung ist bevorzugt ein Steuermittel vorgesehen, beispielsweise ein Microprozessor oder ASIC.

Gemäß einem weiteren Gesichtspunkt der Erfindung wird auch ein Verfahren zum Steuern einer dosierten Verabreichung einer zu injizierenden Flüssigkeit, bevorzugt einer einen medizinischen oder therapeutischen Wirkstoff enthaltenden Flüssigkeit, geschaffen. Bei dem Verfahren wird die Flüssigkeit am Einlass der Ausschüttkontrollvorrichtung mit Druck beaufschlagt. Die Ausschüttkontrollvorrichtung wird betätigt, um eine Ausschüttung der druckbeaufschlagten Flüssigkeit zu bewirken, wobei die Druckbeaufschlagung der Flüssigkeit von der Betätigung der Ausschüttkontrollvorrichtung entkoppelt ist. Das erfindungsgemäße Steuerverfahren kann universell für verschiedene Flüssigkeits- bzw. Medikamentenbehälter, Flüssigkeitsfördermittel und Ausschüttkontrollvorrichtungen verwendet werden. Wichtige Steuerparameter, wie beispielsweise die Zeitdauer, während der die Ausschüttkontollvorrichtung eine Ausschüttung von Flüssigkeit freigibt, der Querschnitt des Auslasses der Ausschüttkontrollvorrichtung während der Flüssigkeitsausschüttung, die Konzentration des in der Flüssigkeit enthaltenen medizinischen oder therapeutischen Wirkstoffs, der am Einlass, der Ausschüttkontrollvorrichtung vorherrschende Überdruck etc., können dem Steuermittel eingegeben werden, beispielsweise von einem Festprogramm oder von in der Infusionspumpe vorgesehenen Sensoren, so dass mit Hilfe des Steuerverfahrens eine präzise Dosierung auch über längere Zeiträume für eine einfache und vergleichsweise kostengünstige Infusionspumpe erzielt werden kann.

Bevorzugt wird das Steuerverfahren als maschinen- oder mikroprozessorlesbares Steuerprogramm realisiert und ist bevorzugt auf einem auch als gesondertes Bauteil erhältlichen Halbleiterbauelement, beispielsweise einem EPROM, einem EEPROM oder dergleichen, gespeichert.

Nachfolgend werden bevorzugte Ausführungsformen der Erfindung unter Bezugnahme auf die beigefügten Figuren beschrieben werden, worin:
- Figur 1: schematisch den Aufbau einer Infusionspumpe gemäß der Erfindung darstellt;
- Figur 2: schematisch den Aufbau einer erfindungsgemäßen Infusionspumpe darstellt, bei der eine Peristaltikpumpe als Ausschüttkontrollvorrichtung dient; und
- Figur 3: schematisch einen Zyklus der in Figur 2 gezeigten Peristaltikpumpe zur Steuerung und Dosierung der ausgeschütteten Flüssigkeitsmenge darstellt.

In den Figuren bezeichnen identische Bezugszeichen identische oder gleichwirkende Bauteile und Funktionsgruppen. Aus den Figuren und der nachfolgenden Beschreibung werden weitere zu lösende Aufgaben, Vorteile und Merkmale der vorliegenden Erfindung dem Fachmann ersichtlich werden.

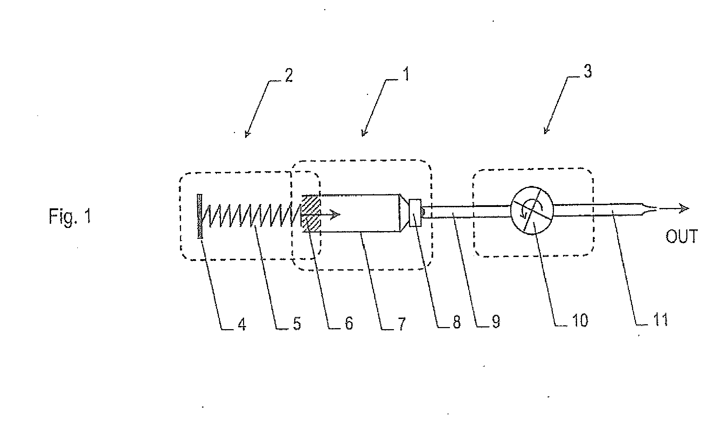

Die Figur 1 zeigt schematisch den Aufbau einer entkoppelten Infusionspumpe gemäß der vorliegenden Erfindung. Die Infusionspumpe umfasst einen Medikamentenbehälter 1, ein Flüssigkeitsfördermittel 2, mittels dem in dem Behälter 1 ein Förderdruck erzeugt wird, und eine Ausschüttkontrollvorrichtung 3, die dem Medikamentenbehälter 1 nachgeordnet ist, um die Ausschüttung der Flüssigkeit aus dem Medikamentenbehälter 1 zu steuern. Der Medikamentenbehälter 1 umfasst eine im Wesentlichen zylindrische oder polygonale, beispielsweise rechteckförmige, Wandung 7, an deren proximalen Ende ein Kolbenstopfen 6 eingesetzt ist, der die in dem Medikamentenbehälter 1 aufbewahrte Flüssigkeit, die einen medizinischen oder therapeutischen Wirkstoff enthält, bei einem axialen Vortrieb desselben durch eine Ausschüttöffnung 8 in die Verbindungsleitung 9 in oder zu der Ausschüttkontrollvorrichtung 3 fördert. Der Medikamentenbehälter 1 kann auch ein Beutel mit zumindest einer flexiblen Seitenwand sein, der zur Ausschüttung der in dem Beutel aufbewahrten Flüssigkeit zusammengedrückt wird, beispielsweise von einem motorgetriebenen oder elektromagnetisch vorgetriebenen Mechanismus, der eine Seitenwand des Beutels einwärts vorschieben kann.

Das Druckerzeugungsmittel 2 umfasst ein Antriebsmittel 5, beispielsweise eine im Ausgangszustand der Infusionspumpe, d.h. in der proximalen Endstellung des Kolbenstopfens 6, maximal vorgespannte Druckfeder, ein vorgespannter Spiralfedermechanismus, der den Kolbenstopfen 6 permanent mit Druck beaufschlagt, ein vorgespannter Drehfedermechanismus, vergleichbar zu dem eines Uhrwerks, der den Kolbenstopfen 6 vortreibt, ein im Ausgangszustand vorgespanntes Elastomer oder eine in dem Ausgangszustand der Infusionspumpe maximal aufgepumpte Pneumatik. Das Antriebsmittel 5 übt auf den Kolbenstopfen 6 eine axial in Richtung auf die Ausschüttöffnung 8 wirkende Antriebskraft, im Ausführungsbeispiel eine Elastizitätskraft, aus. Die Verbindungsleitung 9 und die Seitenwandung 7 des Medikamentenbehälters 1 sind bevorzugt unnachgiebig. Die in dem Medikamentenbehälter 1 und der Verbindungsleitung 9 enthaltene inkompressible Flüssigkeit kann nur dann von dem Kolbenstopfen 6 aus einem Auslass 11 der Ausschüttkontrollvorrichtung 3 ausgeschüttet werden, wenn der Auslass 11 der Ausschüttkontrollvorrichtung 3 geöffnet ist. Bei der in Figur 1 dargestellten Ausführungsform, bei der an dem mit dem Behälter 1 verbundenen Einlass der Ausschüttkontrollvorrichtung 3 permanent ein nicht notwendigerweise konstanter Überdruck besteht, sind das Flüssigkeitsfördermittel 2 und die Ausschüttkontrollvorrichtung 3 voneinander entkoppelt. Ausschüttkontrollvorrichtung 3 und Flüssigkeitsfördermittel 2 können unabhängig voneinander betätigt, manipuliert oder ausgetauscht werden, ohne dass dies einen wesentlichen Einfluss auf die Betriebsweise der Infusionspumpe hat.

Die Ausschüttkontrollvorrichtung 3 dient ausschließlich der Dosierung der Ausschüttung der Flüssigkeit und hat in keiner Weise einen Einfluss auf das Flüssigkeitsförder- bzw. Druckerzeugungsmittel 2.

Die in Figur 1 dargestellte Infusionspumpe zeichnet sich durch einen modularen Aufbau aus, bei dem die wichtigsten Einzelkomponenten, wie beispielsweise Ausschüttkontrollvorrichtung 3, Medikamentenbehälter 1 und Flüssigkeitsfördermittel 2, in einfacher Weise ausgetauscht und durch andere Bauteile ersetzt werden können, beispielsweise in Anpassung an konkrete Einsatzzwecke. Insbesondere kann der Medikamentenbehälter 1 durch einen solchen anderer Größe, anderer Form etc. ersetzt werden, solange das Flüssigkeitsfördermittel 2 die bestimmungsgemäße Funktion ausführen kann, beispielsweise den Kolbenstopfen 6 permanent oder zumindest unmittelbar vor und während der Auslass 11 der Ausschüttkontrollvorrichtung 3 geöffnet ist, mit Druck zu beaufschlagen. Hierzu kann auf der Rückseite des Kolbenstopfens 6 ein universeller Befestigungspunkt zur Verbindung des Kolbenstopfens 6 mit dem Antriebsmittel 5 vorgesehen sein, beispielsweise ein Universalgewinde zur wahlweisen Verbindung mit unterschiedlichen Antriebsgliedern 5, beispielsweise einer Druckfeder, einem Drehfedermechanismus, einem Spiralfedermechanismus, einem vorgespannten Elastomer, einer Pneumatikkammer oder einer Gewindestange eines motorgetriebenen konventionellen Kolbenstopfenvortriebs.

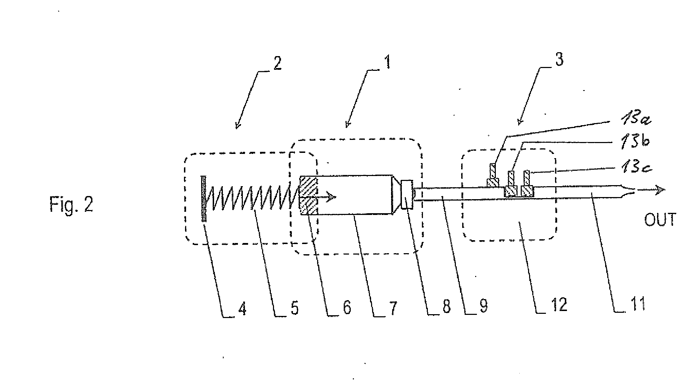

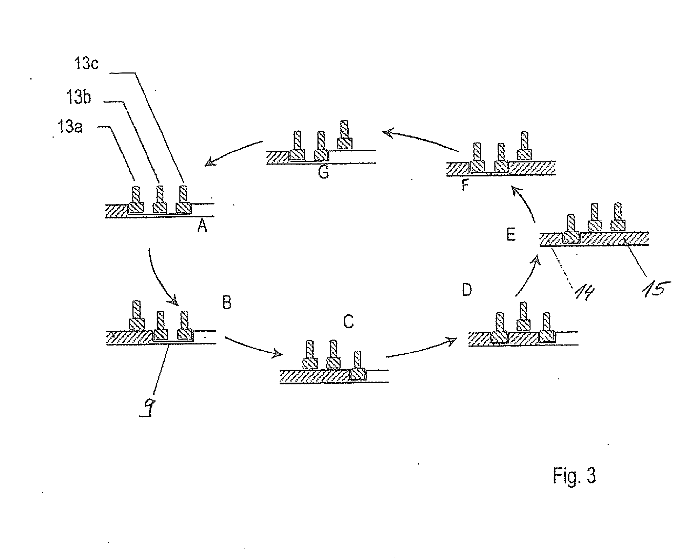

Als Ausschüttkontrollvorrichtung 3 kommt jede Vorrichtung in Frage, die trotz eines permanenten und/oder variablen Überdrucks an ihrem Einlass nur eine genau definierte Menge an Flüssigkeit durchlässt. Beispiele für Ausschüttkontrollvorrichtungen 3 sind ein Schließventil, das zusammen mit einem Bauteil mit definiertem Durchflusswiderstand die abgegebene Flüssigkeitsmenge über die Zeit dosiert, während der das Schließventil geöffnet ist. Ein weiteres Beispiel ist eine im Ruhezustand dicht schließende Durchflusspumpe, beispielsweise die in den Figuren 2 und 3 gezeigte Peristaltikpumpe. Ein weiteres Beispiel ist eine modifizierte Karussell- oder Rollenquetschpumpe, die einen Schlauch permanent und vollständig quetscht, so dass keine Flüssigkeit ausgeschüttet werden kann, und die zur Ausschüttung einen Schlauch weniger stark quetscht, so dass ein vorgebbarer Schlauchquerschnitt die Flüssigkeitsausschüttung freigibt. Ein weiteres Beispiel ist eine im Ruhezustand nicht dichtschließende Durchflusspumpe, beispielsweise eine Diffusorpumpe, die zusammen mit einem Ventil arbeitet, das der Durchflusspumpe vor- oder nachgeordnet ist und das im Ruhezustand einen Auslass permanent versperrt und nur zur Bewirkung der Flüssigkeitsausschüttung geöffnet wird.

Die Ausschüttkontrollvorrichtung 3 unterscheidet sich somit grundsätzlich hinsichtlich einer passiven Betriebsweise, bei der der Einlass der Ausschüttkontrollvorrichtung 3 permanent oder quasi kontinuierlich mit Druck beaufschlagt wird und ein Sehließmittel, beispielsweise ein Ventil, eine Düse oder eine Querschnittsverengung, getaktet zur Flüssigkeitsausschüttung geöffnet wird, und einer aktiven Betriebsweise, bei der das Flüssigkeitsfördermittel lediglich für einen ausreichenden Nachschub der zu verabreichenden Flüssigkeit sorgt und die Ausschüttung aktiv von der Ausschüttkontrollvorrichtung 3 vorgenommen wird, beispielsweise mittels einer in den Figuren 2 und 3 beschriebenen Peristaltikpumpe, einer Karussellpumpe oder dergleichen, die eine vorgebbare Flüssigkeitsmenge durch den Auslass 11 fördert.

Erfindungsgemäß ist die Genauigkeit der Dosierung nicht durch das Flüssigkeitsfordermittel 2 beschränkt, sondern vielmehr durch die Dosiergenauigkeit der Ausschüttkontrollvorrichtung 3 vorgegeben. Es kann deshalb ein einfaches und kostengünstiges Flüssigkeitsfördermittel 2 verwendet werden, das weite Fertigungstoleranzen aufweisen kann. Die erfindungsgemäße Infusionspumpe kann deshalb insbesondere einen vergleichsweise klein ausgebildeten Ausstoßmechanismus umfassen, bei dem Fertigungstoleranzen eine umso größere Rolle spielen. Insbesondere kann zur Herstellung des Ausstoßmechanismus auf die Verwendung von mechanischen Präzisionsbauelementen, beispielsweise präzisen Gewindestangen und dergleichen, verzichtet werden.

Zur Steuerung der Ausschüttkontrollvorrichtung 3 kann ein Steuerprogramm, beispielsweise in einem Halbleiterbauelement integriert, vorgesehen sein, dem wichtige Parameter der dargestellten Infusionspumpe vorliegen oder eingegeben werden können und das die Ausschüttkontrollvorrichtung 3 in geeigneter Weise steuert, um eine gewünschte Dosierung zu erzielen. Wichtige Steuerparameter sind insbesondere die Antriebskraft, mit der der Kolbenstopfen 6 zum Vortrieb permanent oder zumindest während der Flüssigkeitsabgabe beaufschlagt wird, der Querschnitt der Verbindungsleitung 9, der Querschnitt des Auslasses 11 und etwaiger nicht dargestellter nachgeordneter Komponenten, wie beispielsweise Ventile und dergleichen, sowie der Strömungsquerschnitt des Auslasses 11 der Ausschüttkontrollvorrichtung 3. Aus der Antriebskraft, mit der der Kolben 6 beaufschlagt wird, lässt sich der am Einlass der Ausschüttkontrollvorrichtung 3 herrschende Überdruck berechnen, aus dem sich in bekannter Weise in Kenntnis der Strömungsquerschnitte der den Flüssigkeitsstrom bestimmenden Elemente auf die erzielbare Durchflussrate zurückrechnen lässt. Aus der Durchflussrate wiederum lässt sich in Kenntnis der Konzentration des in der Flüssigkeit enthaltenen medizinischen oder therapeutischen Wirkstoffs auf die tatsächlich verabreichte Wirkstoffdosis rückrechnen. Geeignete Rechen- und Steueralgorithmen sind dem Fachmann nach dem Studium der vorliegenden Beschreibung ohne Weiteres ersichtlich und bedürfen deshalb keiner weiteren Ausführungen.

Wichtige Parameter für das Steuerprogramm, beispielsweise die Antriebskraft, mit der der Kolbenstopfen 6 beaufschlagt wird, der am Einlass der Ausschüttkontrollvorrichtung 3 vorherrschende Überdruck etc., können mit dem Fachmann bekannten Sensoren, die an geeigneten Stellen in der Infusionspumpe angeordnet sein können, bestimmt werden und dem Steuerprogramm eingegeben werden, um daraus eine geeignete Zeitablaufsteuerung der Ausschüttkontrollvorrichtung 3 zu berechnen.

Die Infusionspumpe eignet sich insbesondere zur Langzeitabgabe von vergleichsweise geringen Dosen zur Dauerbehandlung von Krankheiten, beispielsweise zur Einstellung des Blutzuckers bei Diabetes-Patienten. Es können somit entsprechend dem Steuerprogramm, dem auch Messsignale eines Sensors zur Blutzuckermessung eingegeben werden können, die Zeitpunkte und zu verabreichenden Dosierungen von in dem Medikamentenbehälter 1 aufbewahrten Insulin vorgegeben werden.

Insbesondere bei Ausführungsformen mit permanenter mechanischer Vorspannung des Kolbenstopfens 6 kann eine besonders energiesparende Infusionspumpe realisiert werden, da der einzige Energieverbraucher im Wesentlichen nur die Ausschüttkontrollvorrichtung 3 und deren Steuerprogramm sind. Das Antriebsmittel 5, beispielsweise eine Rückstellfeder, kann mechanisch, beispielsweise von Hand, vor Inbetriebnahme der Infusionspumpe vorgespannt werden. Während des Betriebs der Infusionspumpe baut das Antriebsmittel 5 unter Erzeugung der Antriebskraft wegen des Vortriebs des Kolbenstopfens 6 seine Vorspannkraft gänzlich oder vorzugsweise nur zu einem Teil ab. Die Figur 2 zeigt eine Variante der in Figur 1 gezeigten Ausführungsform, bei der die Ausschüttkontrollvorrichtung 3 als Peristaltikpumpe 12 realisiert ist. Die Peristaltikpumpe 12 umfasst, drei Finger 13a, 13b und 13c, die beispielsweise von nicht dargestellten Nocken und einer Drehwelle angetrieben werden können, um eine im Wesentlichen zur Strömungsrichtung vertikale Bewegung auszuführen zwischen einer oberen Stellung, bei der ein Querschnitt der Verbindungsleitung 9 freigegeben wird und einer unteren Stellung, bei der der Querschnitt der Verbindungsleitung 9 bevorzugt vollständig gesperrt wird, so dass keine Flüssigkeit an den Auslass 11 der Peristaltikpumpe 12 weitergegeben werden kann.

Die Peristaltikpumpe 12 führt den schematisch in der Figur 3 dargestellten Zyklus aus, der beim Ausgangszustand A beginnt, bei dem sämtliche Finger 13a-c den Querschnitt der Verbindungsleitung 9 so sperren, dass das stromaufwärtige Flüssigkeitsvolumen 14, stromaufwärts des hinteren Fingers 13a, gesperrt ist. Anschließend wird der hintere Finger 13a in die obere Endstellung angehoben, so dass das stromaufwärtige Flüssigkeitsvolumen 14 bis zu dem mittleren Finger 13b fortschreitet. Durch Anheben des mittleren Fingers 13b wird das stromaufwärtige Flüssigkeitsvolumen 14 weiter bis zu dem stromabwärtigen, vorderen Finger 13c weiterbefördert. Anschließend wird der hintere Finger 13a in die untere Endstellung gebracht, so dass ein Teil des stromaufwärtigen Flüssigkeitsvolumens 14 zwischen dem vorderen Finger 13a und dem hinteren Finger 13c eingeschlossen ist. Durch Wahl der Hubhöhe des mittleren Fingers 13b kann die tatsächlich verabreichte Dosis vorgegeben werden, falls der mittlere Finger 13b zwischen seinen beiden Einstellungen zusätzlich auch Zwischenstellungen einnehmen und halten kann. Nach Öffnen des vorderen Fingers 13c (Schritt E) und Überführen des mittleren Fingers 13b in die untere Endstellung (Schritt F) und Überführen des hinteren Ringers 13c in die untere Endstellung (Schritt F) wird schließlich wieder der Ausgangszustand A erreicht, wobei eine mittels der Ausschüttkontrollvorrichtung 3 vorgegebene Flüssigkeitsmenge an das stromabwertige Flüssigkeitsvolumen 15 abgegeben wurde.

Bei dieser Variante wird das stromaufwärtige Flüssigkeitsvolumen 14 durch den am Einlass der Peristaltikpumpe 12 vorherrschenden Überdruck weitergefördert in die Peristaltikpumpe 12, von wo aus die kontrollierte Flüssigkeitsabgabe unter Steuerung des vorgenannten Steuerprogramms erfolgt.

Durch Änderung der Steuerparameter des vorgenannten Steuerprogramms kann die Flüssigkeitsabgabe variabel und für eine Vielzahl unterschiedlicher Komponenten der Infusionspumpe gestaltet werden, was dem Fachmann beim Studium dieser Patentbeschreibung ohne Weiteres ersichtlich sein wird. Die Flüssigkeitsabgabe kann nach einem vorprogrammierten Profil, beispielsweise eingegeben von dem zu behandelnden Arzt und/oder gemäß den Wünschen des Patienten, erfolgen. Die Infusionspumpe kann mit einem austauschbaren Medikamentenbehälter betrieben werden. Besonders vorteilhaft ist die vorstehend beschriebene Infusionspumpe als modulares Infusionspumpensystem verwendbar, bei dem eine identisch ausgestaltete Ausschüttkontrollvorrichtung für beliebige Medikamentenmodule, d.h. bestehend aus Ausschlussmechanismus und Medikamentenbehälter, verwendet werden kann.

## Patentansprüche

1. Infusionspumpe zur dosierten Verabreichung einer zu injizierenden medizinischen Flüssigkeit, umfassend
a) einen Flüssigkeitsbehälter (1) zur Aufbewahrung der Flüssigkeit,
b) ein Flüssigkeitsfördermittel (2) zum Fördern der Flüssigkeit aus dem die zu injizierende Flüssigkeit enthaltenden Flüssigkeitsbehälter (1)
c) und eine Ausschüttkontrollvorrichtung (3) zur Dosierung der Ausschüttung der Flüssigkeit,
d) wobei das Flüssigkeitsfördermittel (2) die in dem Flüssigkeitsbehälter (1) aufbewahrte Flüssigkeit mit Druck beaufschlagt, so dass die Flüssigkeit von einer Ausschüttöffnung (8) des Flüssigkeitsbehälters (1) zu einem Einlass (9) der Ausschüttkontrollvorrichtung (3) gefördert wird,
e) das Flüssigkeitsfördermittel (2) die Flüssigkeit am Einlass (9) der Ausschüttkontrollvorrichtung (3) mit Druck beaufschlagt,
f) das Flüssigkeitsfördermittel (2) von der Ausschüttkontrollvorrichtung (3) entkoppelt ist,
g) der Flüssigkeitsbehälter (1) einen Kolbenstopfen (6) umfasst, der die Flüssigkeit bei seinem Vortrieb ausschüttet,
h) und das Flüssigkeitsfördermittel (2) den Kolbenstopfen (6) vortreibt.

2. Infusionspumpe nach Anspruch 1, bei der das Flüssigkeitsfördermittel (2) die Flüssigkeit am Einlass (9) der Ausschüttkontrollvorrichtung (3) permanent mit Druck beaufschlagt.

3. Infusionspumpe nach einem der vorhergehenden Ansprüche, bei der das Flüssigkeitsfördermittel (2) den Flüssigkeitsbehälter (1) permanent mit Druck beaufschlagt, so dass die Flüssigkeit unkontrolliert zu der Ausschüttkontrollvorrichtung (3) gefördert wird.

4. Infusionspumpe nach einem der vorhergehenden Ansprüche, bei der das Flüssigkeitsfördermittel (2) den Flüssigkeitsbehälter (1) an einem der Ausschüttöffnung (8) gegenüberliegenden Ende des Flüssigkeitsbehälters mit Druck beaufschlagt.

5. Infusionspumpe nach einem der vorhergehenden Ansprüche, bei der das Flüssigkeitsfördermittel (2) ein Antriebsmittel (5) umfasst, um den Kolbenstopfen (6) hin zu der Ausschüttöffnung (8) mit einer Antriebskraft zu beaufschlagen.

6. Infusionspumpe nach Anspruch 5, bei der das Antriebstellmittel ein motorischer Kolbenstopfenvortrieb, vorzugsweise mit geregelter Stoßkraft, zum Vortrieb des Kolbenstopfens (6) ist.

7. Infusionspumpe nach Anspruch 5 oder 6, bei der das Antriebsmittel ein Spiralfedermechanismus, ein Drehfedermechanismus, ein Elastomer, ein gekapseltes Druckgasreservoir oder eine aufpumpbare Pneumatik ist.

8. Infusionspumpe nach einem der vorhergehenden Ansprüche, bei der das Flüssigkeitsfördermittel (2) getaktet ist, um den Einlass (9) der Ausschüttkontrollvorrichtung zumindest vor und während einer Betätigung der Ausschüttkontrollvorrichtung (3) zur Ausschüttung mit Druck zu beaufschlagen.

9. Infusionspumpe nach einem der vorhergehenden Ansprüche, bei der die Flüssigkeit an dem Einlass (9) der Ausschüttkontrollvorrichtung (3) mit einem variablen Druck beaufschlagbar ist.

10. Infusionspumpe nach einem der vorhergehenden Ansprüche, wobei die Ausschüttkontrollvorrichtung (3) in einem Ruhezustand schließt und ein Steuermittel vorgesehen ist, um die Ausschüttung einer dosierten Flüssigkeitsmenge zu bewirken.

11. Infusionspumpe nach einem der vorhergehenden Ansprüche, bei der die Ausschüttkontrollvorrichtung ein Schließventil und ein Bauteil mit definiertem Durchflusswiderstand umfasst, um eine über die Zeit, während der das Ventil offen ist, dosierte Flüssigkeitsmenge abzugeben.

12. Infusionspumpe nach einem der Ansprüche 1 bis 10 und wenigstens einem der folgenden Merkmale:
(i) die Ausschüttkontrollvorrichtung umfasst eine im Ruhezustand dicht schließende Durchflusspumpe, insbesondere Peristaltikpump;
(ii) die Ausschüttkontrollvorrichtung umfasst eine Karussell- oder Rollenquetschpumpe (12).

13. Infusionspumpe nach einem der Ansprüche 1 bis 10, bei der die Ausschüttkontrollvorrichtung (3) eine im Ruhezustand nicht dicht schließende Durchflusspumpe, insbesondere eine Diffusorpumpe, ist, der ein Schließventil nachgeordnet ist.

14. Infusionspumpe nach einem der vorhergehenden Ansprüche, wobei der Querschnitt des Auslasses der Ausschüttkontrollvorrichtung (3) variabel vorgebbar ist.

15. Infusionspumpe nach einem der vorhergehenden Ansprüche, umfassend ein Steuermittel, auf dem Programmcodemittel ablaufen, die bewirken, dass die Flüssigkeit am Einlass (9) der Ausschüttkontrollvorrichtung (3) mit Druck beaufschlagt wird und die Ausschüttkontrollvorrichtung (3) betätigt wird, um eine Ausschüttung der druckbeaufschlagten Flüssigkeit zu bewirken, wobei die Druckbeaufschlagung der Flüssigkeit von der Betätigung der Ausschüttkontrollvorrichtung (3) entkoppelt ist.
